# EUROPÄISCHE PATENTSCHRIFT

(11) **EP 1 457 475 B1**
(45) Veröffentlichungstag und Bekanntmachung des Hinweises auf die Patenterteilung: **30.12.2009**
(21) Anmeldenummer: 04002145.3
(22) Anmeldetag: 31.01.2004
(51) Int. Cl.: C07C 2/10

(54) **Verfahren zur Herstellung von Oligomeren von Alkenen mit 4 bis 8 Kohlenstoffatomen**
Process for the synthesis of oligomers of alkenes with 4 to 8 carbon atoms
Procédé pour la production d'oligomères d'alkènes contenant 4 à 8 atomes de carbone

(30) Priorität: 11.03.2003 DE 10310483
(43) Veröffentlichungstag der Anmeldung: 15.09.2004
(73) Patentinhaber: BASF SE, 67056 Ludwigshafen (DE)
(72) Erfinder: Heidemann, Thomas, 68519 Viernheim (DE); Ulonska, Armin, 67150 Niederkirchen (DE); Stäck, Bianca, 67117 Limburgerhof (DE)

(56) Entgegenhaltungen:
- WO-A-00/69795
- WO-A-01/72670
- WO-A-99/25668

## Beschreibung

Die vorliegende Erfindung betrifft ein Verfahren zur Herstellung von Oligomeren von Alkenen mit 4 bis 8 Kohlenstoffatomen ausgehend von einem Feed-Strom aus solchen Alkenen oder solche Alkene enthaltenden Kohlenwasserstoffströmen an einem Nickel-haltigen, heterogenen Katalysator in n aufeinander folgenden adiabatisch betriebenen Reaktoren, wobei n für 2 oder eine ganze Zahl größer 2 steht, bei Temperaturen von 30 bis 280°C und Drücken von 1 bis 300 bar und wobei der Feed-Strom mit einer Temperatur Tₑᵢₙ in die erste Reaktionszone eintritt, in jeder Reaktionszone eine Temperaturerhöhung erfährt und, falls diese Temperaturerhöhung mehr als Tₑᵢₙ + 20°C beträgt, vor Eintritt in eine nachfolgende Reaktionszone auf eine Temperatur im Intervall Tₑᵢₙ± 20°C eingestellt wird.

Alkene mit 2 bis 8 Kohlenstoffatomen oder deren Gemische stehen in großen Mengen sowohl aus FCC-Anlagen (Fluidized Catalyst Cracking) als auch aus Steamcrackern zur Verfügung. Es ist bekannt, die C₄-Fraktion, d.h. ein im Wesentlichen aus Butenen und Butanen bestehendes Gemisch, gegebenenfalls nach Abtrennung des iso-Butens, zur Herstellung von Oligomeren, insbesondere von Octenen und Dodecenen, zu verwenden. Sowohl die Octene als auch die Dodecene können nach der Hydroformylierung und anschließender Hydrierung zu den entsprechenden Alkoholen z.B. zur Herstellung von Weichmachern oder Tensiden verwendet werden.

Die Oligomerisierung wird großtechnisch entweder unter homogener oder unter heterogener Katalyse durchgeführt. Die wichtigsten industriell ausgeübten heterogen katalysierten Alken-Oligomerisierungsverfahren werden z.B. in A. Chauvel und G. Lefebvre, Petrochemical Process, Edition Technip (1989), S. 183-187 und F. Asinger, "Die petrolchemische Industrie", Akademie-Verlag (1971), S. 278-299 dargestellt.

Die Oligomerisierung von Alkenen verläuft am heterogenen Katalysator normalerweise exotherm. Die Temperatur des Reaktionsgemischs steigt beim Durchgang durch die Reaktionszone somit in der Regel allmählich an. Wegen der damit verbundenen niedrigeren Investitionskosten wird der Oligomerisierung in adiabatisch betriebenen Reaktoren häufig der Vorzug gegeben.

Die Katalysatoraktivität nimmt bei solchen Reaktionen häufig mit der Zeit, d.h. mit der verstrichenen produktiven Betriebszeit, ab. Es scheint insbesondere eine Proportionalität zwischen der in Produktivbetrieb am Katalysator entwickelten Reaktionswärme und der Abnahme der Katalysatoraktivität zu bestehen.

lässt die Katalysatoraktivität in der Oligomerisierungs-Reaktionszone normalerweise nach, was teilweise durch ein Anheben der Temperatur am Katalysator ausgeglichen werden kann.

Zur Temperaturkontrolle in der Reaktionszone kann die Anfangstemperatur des Reaktionsgemischs herangezogen werden. Sie muss ausreichend hoch sein, um die gewünschte Katalysatoraktivität und Reaktionsgeschwindigkeit zu erreichen, welche in der Regel den Umsatz maßgeblich beeinflussen. Im Übrigen wird man aus Gründen der Energieersparnis einer Eintrittstemperaturdie in der Nähe der Umgebungstemperatur den Vorzug geben.

Ist die zulässige Maximaltemperatur erreicht, kann die Aktivität des Katalysators üblicherweise allein nicht weiter über die Temperatur gesteigert werden; die Temperatur des Reaktionsgemischs ist darüber hinaus in der Praxis häufig durch den zulässigen Maximaldruck des Reaktors nach oben hin begrenzt.

Aus der WO-A 01/72670 ist ein Verfahren zur Oligomerisierung von Alkenen bekannt, bei welchem man den Austrag aus dem quasi-isotherm betriebenen Reaktor teilweise auf das Endprodukt aufarbeitet und den anderen - nicht aufgearbeiteten - Teil, zusammen mit frischem Feed, in den Reaktor zurückführt. Durch den fehlenden Aufarbeitungsschritt kommt es im Feed-Strom allerdings zu einer Aufpegelung von Nebenprodukten, insbesondere von Oligomeren.

In der WO-A 00/69795 wird ein Verfahren zur Oligomerisierung von Alkenen beschrieben, bei welchem das Volumen der einzelnen Reaktionszonen so eingestellt wird, dass sich die Austrittstemperaturen der Reaktionsgemische aus zwei unterschiedlichen Reaktionszonen um höchstens 20°C unterscheidet. Durch die Temperatursteuerung, die anhand der Reaktorvolumina erfolgt, ist dieses Verfahren jedoch apparativ auf eine ganz bestimmte Fahrweise festgelegt.

Die Dauer, die ein bestimmter Katalysator produktiv verwendet werden kann, bestimmt bei den vorgestellten Verfahren maßgeblich die Wirtschaftlichkeit des gesamten Verfahrens, so dass auf Seiten der chemischen Industrie ein fortwährendes Interesse daran besteht, Katalysatoren mit verlängerter Lebensdauer und diesen Katalysatoren angepasste verbesserte Verfahren zu entwickeln.

Der vorliegenden Erfindung lag demnach ein Verfahren zur Oligomerisierung von Alkenen zu Grunde, bei dem sich durch eine verbesserte Feed-Verwendung die Katalysator-Standzeit verlängert.

Demgemäß wurde ein Verfahren zur Herstellung von Oligomeren von Alkenen mit 4 bis 8 Kohlenstoffatomen ausgehend von einem Feed-Strom aus solchen Alkenen oder solche Alkene enthaltenden Kohlenwasserstoff strömen an einem Nickel-haltigen, heterogenen Katalysator in n aufeinander folgenden adiabatisch betriebenen Reaktoren gefunden, wobei n für 2 oder eine ganze Zahl größer 2 steht, bei Temperaturen von 30 bis 280°C und Drücken von 1 bis 300 bar und wobei der Feed-Strom mit einer Temperatur Tₑᵢₙ in die erste Reaktionszone eintritt, in jeder Reaktionszone eine Temperaturerhöhung erfährt und, falls diese Temperaturerhöhung mehr als Tₑᵢₙ + 20°C beträgt, vor Eintritt in eine nachfolgende Reaktionszone auf eine Temperatur im Intervall Tₑᵢₙ± 20°C eingestellt wird, welches sich dadurch auszeichnet, dass der Feed-Strom aufgeteilt und die so erhaltenen Feed-Teilströme den beiden oder bei Verwendung von mehr als 2 Reaktoren mindestens 2 der Reaktoren unter Zusatz von frischem Feed so zugeführt werden, dass die Temperatur in einem der Reaktoren maximal 20°C höher ist als die in jedem der anderen verwendeten Reaktoren.

Unter "Oligomeren" werden hierin Dimere, Trimere und höhere oligomere Produkte der Umsetzung von Alkenen mit 4 bis 8 Kohlenstoffatomen mit bis zu 18, vorzugsweise bis zu 12 und besonders bevorzugt bis zu 8 Kohlenstoffatomen verstanden.

"Feed" wird hierin als Sammelbegriff für reaktive Alkene enthaltende Ausgangsstoffe verwendet, welche den Reaktoren zugeführt werden.

Als "Katalysator-Standzeit" wird die gesamte Verwendungsdauer eines Katalysators bei der Oligomerisierung von Alkenen bezeichnet. Während dieser Verwendungsdauer nimmt die katalytische Aktivität in der Regel ab. Ein Austausch des Katalysators erfolgt normalerweise bei einer katalytischen Restaktivität, bei der das Verfahren nicht mehr wirtschaftlich ist.

Unter einer "adiabatischen Reaktionsführung" wird im technischen Sinne eine Reaktionsführung verstanden, bei der, abgesehen von dem durch natürliche Wärmeleitung und Wärmeabstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird.

Unter den Alkenen mit 2 bis 8 Kohlenstoffatomen, die sich mit dem erfindungsgemäßen Verfahren zu Oligomeren umsetzen lassen sind die einfach ungesättigten Butene, Pentene und Hexene bevorzugt. Besonders bevorzugt sind für sich allein 1-Buten, 2-Buten, 1-Penten, 2-Penten, 1-Hexen oder 3-Hexen.

Für das erfindungsgemäße Verfahren eignen sich Feeds, deren Gehalt an Alken oder Alkenen normalerweise bei 5 bis 100, vorzugsweise bei 30 bis 100 und besonders bevorzugt bei 50 bis 100 Gew.-% liegt.

Ganz besonders geeignet ist das erfindungsgemäße Verfahren für die Umsetzung von Alkenen, die im Gemisch mit Alkanen vorliegen, wobei Alkene und Alkane überwiegend insbesondere jeweils 4 Kohlenstoffatome aufweisen. Geeignete derartige C₄-Kohlenwasserströme sind z.B. Gemische mit folgender Zusammensetzung:

| | |
|---|---|
| Butane | 10 bis 90 Gew.-% |
| Butene | 10 bis 90 Gew.-%, |

wobei die Butene-Fraktion folgende Zusammensetzung haben kann:

| | |
|---|---|
| 1-Buten | 1 bis 50 Gew.-% |
| cis-2-Buten | 1 bis 50 Gew.-% |
| trans-2-Buten | 1 bis 99 Gew.-% |
| iso-Buten | 1 bis 5 Gew.-%. |

Als besonders bevorzugter Feed wird das sogenannte Raffinat II verwendet. Es handelt sich dabei um ein Buten-haltiges C₄-Kohlenwasserstoffgemisch, wie es aus dem C₄-Schnitt von Crackern erhalten wird durch Abtrennen von höher ungesättigten Kohlenwasserstoffen, wie Dialkenen, insbesondere 1,3-Butadien, oder Acetylen und anschließend iso-Buten. Eine typische Zusammensetzung für Raffinat II ist:

| | |
|---|---|
| iso-, n-Butan | 26 Gew.-% |
| iso-Buten | 1 Gew.-% |
| 1-Buten | 26 Gew.-% |
| trans-2-Buten | 31 Gew.-% |
| cis-2-Buten | 16 Gew.-%. |

Die Feeds können vor der Umsetzung gemäß der DE-A 39 14 817 durch Hydrierung bzw. Absorption an Molekularsieb von Butadien, schwefelhaltigen und sauerstoffhaltigen Verbindungen, wie Alkoholen, Aldehyden, Ketonen oder Ethern befreit werden.

Als Katalysatoren kommen Nickel-haltige heterogene Katalysatoren zum Einsatz, vorzugsweise mit einer hohen Selektivität hinsichtlich linearer Oligomeren. Derartige Nickel-haltigen Katalysatoren sind dem Fachmann etwa aus der WO-A 01/72670 bekannt.

Die Ni-Katalysatoren werden vorzugsweise in einem Festbett angeordnet und haben vorzugsweise stückige Form: z.B. Tabletten (5 mm x 5 mm, 5 mm x 3 mm, 3 mm x 3 mm), Ringe (7 mm x 7 mm x 3 mm, 5 mm x 5 mm x 2 mm, 5 mm x 2 mm x 2 mm) oder Stränge bzw. Sternenstränge (1,5 mm-Durchmesser, 3 mm-Durchmesser, 5 mm-Durchmesser). Die vorstehenden Größenangaben und Formkörpertypen sind lediglich beispielhaft und stellen keine Einschränkung des Gegenstandes der vorliegenden Erfindung dar.

In den einzelnen Reaktoren der Reaktorkaskade können gleiche oder unterschiedliche Nickel-haltige Katalysatoren eingesetzt werden.

Als Reaktoren dienen normalerweise mit dem Nickel-haltigen Katalysator beschickte zylindrische Reaktoren ohne zusätzliche Einbauten.

Die Oligomerisierungsreaktion findet in der Regel bei Temperaturen von 30 bis 280°C, vorzugsweise von 30 bis 140°C und insbesondere von 40 bis 130°C und einem Druck von in der Regel 1 bis 300 bar, vorzugsweise von 5 bis 100 bar und insbesondere von 20 bis 70 bar statt. Der Druck wird dabei zweckmäßigerweise so ausgewählt, dass der Feed-Strom bei der eingestellten Temperatur überkritisch und insbesondere flüssig vorliegt.

In den einzelnen Reaktoren der Reaktorkaskade können unterschiedliche Reaktionsbedingungen hinsichtlich Druck und/oder Temperatur im Rahmen der obengenannten Druck- und Temperaturbereiche eingestellt werden.

Nach dem Verlassen eines Reaktors wird der jeweilige rohe Produktstrom in an sich bekannter Weise, vorzugsweise destillativ, auf die gebildeten Oligomeren aufgearbeitet. Der dabei anfallende Reststrom von nicht umgesetzten Alkenen und gegebenenfalls begleitenden Alkanen wird - als Rückführstrom - teilweise in den selbigen Reaktor zurückgeführt und im Übrigen - zusammen mit dem benötigten Teilstrom von frischem Feed - in den nachfolgenden Reaktor geleitet.

Nach Umsetzung im letzten Reaktor liegt der Gehalt an Alkenen im Reststrom in der Regel zwischen 5 bis 20 Gew.-%; der Reststrom wird teilweise als Rückführstrom weiterverwendet und im Übrigen aus dem Prozess ausgeschleußt.

Restströme (Rückführströme) und Feed-Teilströme können vor dem Einleiten in einen Reaktor mit hierzu üblichen Vorrichtungen wie Wärmetauschern auf die gewünschte Temperatur gestellt werden.

In welchem Verhältnis der Strom des frischen Feeds und des jeweiligen Reststroms in den nachfolgenden Reaktor geleitet werden, kann der Fachmann im Hinblick auf die einzustellende Temperatur in diesem nachfolgenden Reaktor leicht anhand einfacher Vorversuche ermitteln.

Feed-Teilstrom und rückgeführter Reststrom können dabei gleichzeitig einzeln, etwa über getrennte Leitungen, oder nach vorherigem Vermischen in den Reaktor geleitet werden.

Der Umsatz zu den gewünschten Oligomeren liegt beim erfindungsgemäßen Verfahren in der Regel bei 10 bis 100, und vorzugsweise bei 50 bis 100 %, bezogen auf die eingesetzten Alkene.

Die Verfahrensführung ist im Übrigen dem Fachmann vor allem aus der WO-A 99/25668, der WO-A 01/72670 und der WO-A 00/69795 geläufig, auf die hiermit diesbezüglich vollinhaltlich Bezug genommen wird.

Das erfindungsgemäße Verfahren hat den Vorteil, dass die Temperaturbelastung an den Katalysatoren in den verschiedenen Reaktoren angeglichen wird. Dadurch verlängert sich regelmäßig die Standzeit derjenigen Katalysatoren, die dadurch bei niedrigerer Temperatur betrieben werden können. Entsprechend kommt es zu vergleichbaren Abnahmen der Katalysatoraktivität in den Reaktoren, so dass ein Katalysatorwechsel zur gleichen Zeit in allen Reaktoren wirtschaftlicher wird.

### Beispiele

Als Feed dienten in den Beispielen 1 und 2 jeweils 8,1 kg/h Raffinat II der Zusammensetzung: 5 Gew.-% iso-Butan, 18 Gew.-% n-Butan, 31 Gew.-% 1-Buten, 2 Gew.-% iso-Buten, 28 Gew.-% trans-2-Buten, 16 Gew.-% cis-2-Buten.

Die Versuche wurden in einer zweistufigen adiabaten Reaktorkaskade an einem Katalysator gemäß DE-A 43 39 713, Beispiel 2 durchgeführt. Der erste Reaktor wies ein Volumen von 40, der zweite von 20 Litern auf. Nach dem ersten und dem zweiten Reaktor wurde gebildetes C₈₊-Alken (Alken mit zwei oder mehr als zwei Buteneinheiten) destillativ von unumgesetztem C₄-Alken bzw. inertem C₄-Alkan abgetrennt. Ein Teil des so abgetrennten C₄-Kohlenwasserstoffstroms wurde - wie in den Beispielen 1 und 2 unten näher spezifiziert wird - weiterverwertet.

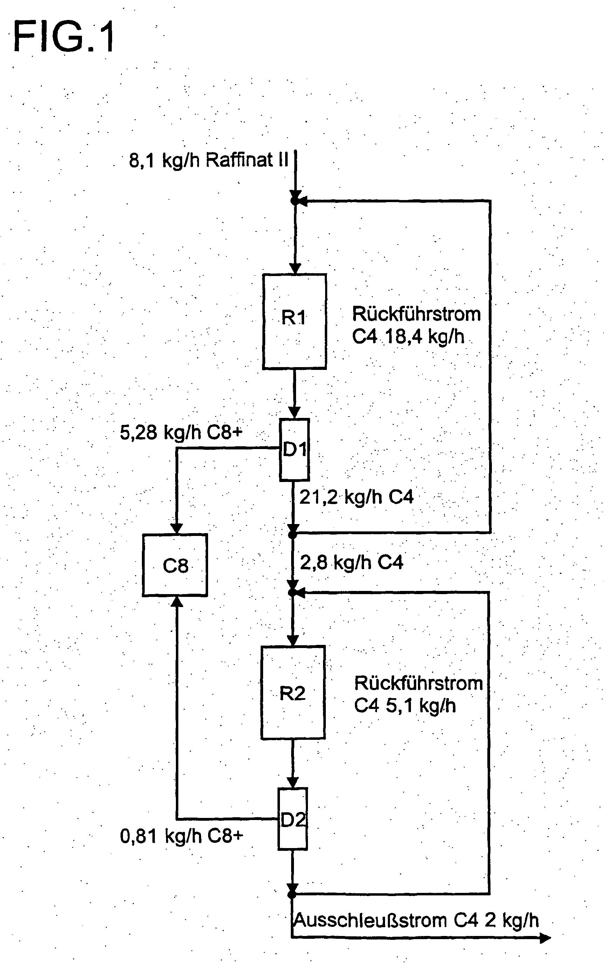

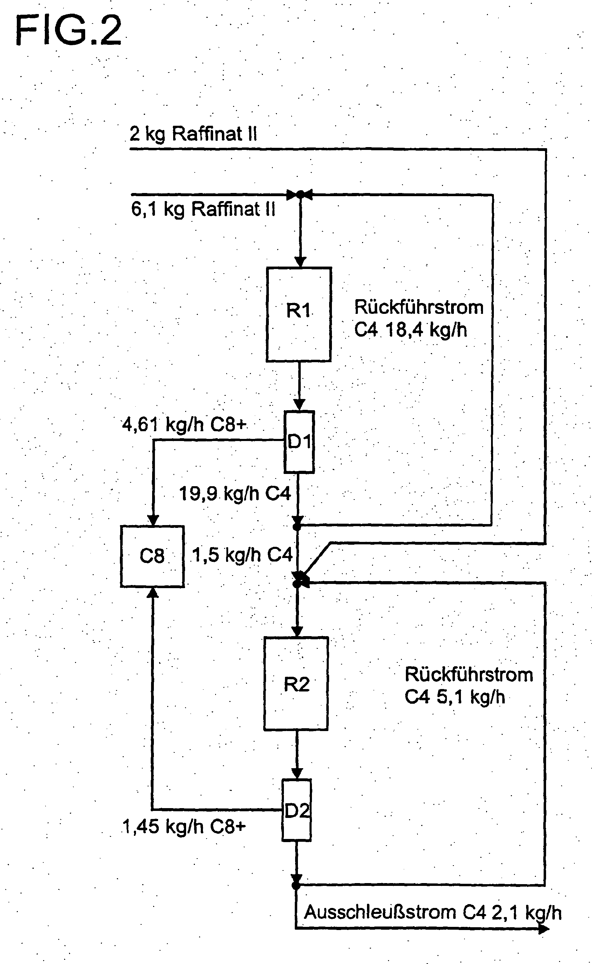

Stoffströme und Vorrichtung von Beispiel 1. und 2 sind in Figur 1 und Figur 2 schematisch dargestellt. Es bedeuten darin:
R1 = Reaktor 1
R 2 = Reaktor 2
D1 = Destillationskolonne 1
D2 = Destillationskolonne 2.

### Beispiel 1 (Vergleichsbeispiel, Figur 1)

8,1 kg/h Raffinat II als Feed wurden mit 18,4 kg/h Rückführstrom aus Reaktor 1 gemischt, bei 20°C Eingangstemperatur in den Reaktor 1 geleitet und dort zur Umsetzung gebracht. Das Reaktionsgemisch erwärmte sich dabei um 54°C auf 74°C am Reaktorausgang.

Der Austrag aus Reaktor 1 wurde destillativ in 5,28 kg/h Sumpfprodukt (>99 Gew.-% C₈₊) und 21,2 kg/h Kopfprodukt (>99 Gew.-% C₄) aufgetrennt. 18,4 kg/h dieses Kopfproduktes bildeten den Rückführstrom zum Reaktor 1.

Die übrigen 2,8 kg des Kopfproduktes wurden mit 5,1 kg Rückführstrom aus Reaktor 2 gemischt, bei 40°C Reaktoreingangstemperatur in den Reaktor 2 geleitet und dort zur Umsetzung gebracht.

Das Reaktionsgemisch erwärmte sich dabei um 25°C auf 65°C am Reaktorausgang.

Der Austrag aus Reaktor 2 wurde destillativ in 0,81 kg/h Sumpfprodukt (>99 Gew.-% C₈₊) und 7,1 kg/h Kopfprodukt aufgetrennt. 5,1 kg dieses Kopfprodukts bildeten den Rückführstrom aus Reaktor 2; die übrigen 2 kg/h wurden aus dem Versuch ausgeschleust.

Eine Analyse der vereinigten Sumpfprodukte ergab eine C₈₊-Selektivität (Sel_{C8+}) von 82 %. Der Gesamtbuteneumsatz (U_{Butene}) betrug 97,9 %.

### Beispiel 2 (erfindungsgemäßes Beispiel, Figur 2)

Die 8,1 kg/h Raffinat II wurden in zwei Ströme zu 6,1 kg/h und 2 kg/h aufgeteilt.

Der Strom mit 6,1 kg/h Raffinat II wurde mit 18,4 kg/h Rückführstrom aus Reaktor 1 gemischt, bei 20°C Eingangstemperatur in den Reaktor 1 geleitet und dort zur Umsetzung gebracht. Das Reaktionsgemisch erwärmte sich dabei um 45°C auf 65°C am Reaktorausgang.

Der Austrag aus Reaktor 1 wurde destillativ in 4,61 kg/h Sumpfprodukt (>99 Gew.-% C₈₊) und 19,9 kg/h Kopfprodukt (>99 Gew.-% C4) aufgetrennt. 18,4 kg/h dieses Kopfproduktes bildeten den Rückführstrom zum Reaktor 1.

Die übrigen 1,5 kg des Kopfproduktes wurden mit den eingangs dieses Beispiels 2 erwähnten 2 kg/h frischen Raffinats II sowie 5,1 kg Rückführstrom aus Reaktor 2 gemischt, bei 25°C Reaktoreingangstemperatur in den Reaktor 2 geleitet und dort zur Umsetzung gebracht.

Das Reaktionsgemisch erwärmte sich dabei um 40°C auf 65°C am Reaktorausgang.

Der Austrag aus Reaktor 2 wurde destillativ in 1,45 kg/h Sumpfprodukt (>99 Gew.-% C₈₊) und 7,2 kg/h Kopfprodukt aufgetrennt. 5,1 kg dieses Kopfprodukts bildeten den Rückführstrom aus Reaktor 2; die übrigen 2,1 kg/h wurden aus dem Versuch ausgeschleußt.

Eine Analyse der vereinigten Sumpfprodukte ergab eine C₈₊-Selektivität Sel_{C8+}von 82 %. Der Gesamtbuteneumsatz U_{Butene} betrug 97,4 %.

### Bewertung der Beispiele 1 und 2

| Beispiel | T_{R1ein} [°C] | T_{R1aus} [°C] | ΔT_{R1aus-R1ein} [°C] | T_{R2ein} [°C] | T_{R2aus} [°C] | ΔT_{R2aus-R2ein} [°C] | U_{Butene} [%] | Sel_{C8+} |
|---|---|---|---|---|---|---|---|---|
| 1 | 20 | 74 | 54 | 40 | 65 | 25 | 97,9 | 82 |
| 2 | 20 | 65 | 45 | 25 | 65 | 40 | 97,4 | 82 |

- T_{R1ein}: Eintrittstemperatur am Reaktor R1
- T_{R1aus}: Austrittstemperatur am Reaktor R1
- ΔT_{R1aus-R1ein}: Differenz von Aus- und Eintrittstemperatur am Reaktor R1
- T_{R2ein}: Eintrittstemperatur am Reaktor R2
- T_{R2aus}: Austrittstemperatur am Reaktor R2
- ΔT_{R2aus-R2ein}: Differenz von Aus- und Eintrittstemperatur am Reaktor R2
- U_{Butene}: Gesamtbuteneumsatz
- Sel_{C8+}: C₈₊-Selektivität

Die Aufteilung des Feeds im erfindungsgemäßen Beispiel 2 führte zu einer Angleichung der Temperaturbelastung an den Katalysatoren in den Reaktoren R1 und R2, was zu einer verlängerten Katalysatorstandzeit führt. Der Umsatz der Butene (U_{Butene}) sowie die C₈₊-Selektivität (Sel_{C8+}) blieben dabei gegenüber der Fahrweise von Beispiel 1 praktisch unverändert.

## Patentansprüche

1. Verfahren zur Herstellung von Oligomeren von Alkenen mit 4 bis 8 Kohlenstoffatomen ausgehend von einem Feed-Strom aus solchen Alkenen oder solche Alkene enthaltenden Kohlenwasserstoffströmen an einem Nickel-haltigen, heterogenen Katalysator in n aufeinander folgenden adiabatisch betriebenen Reaktoren, wobei n für 2 oder eine ganze Zahl größer 2 steht, bei Temperaturen von 30 bis 280°C und Drücken von 1 bis 300 bar und wobei der Feed-Strom mit einer Temperatur Tₑᵢₙ in die erste Reaktionszone eintritt, in jeder Reaktionszone eine Temperaturerhöhung erfährt und, falls diese Temperaturerhöhung mehr als Tₑᵢₙ + 20°C beträgt, vor Eintritt in eine nachfolgende Reaktionszone auf eine Temperatur im Intervall Tₑᵢₙ± 20°C eingestellt wird, **dadurch gekennzeichnet, dass** der Feed-Strom aufgeteilt und die so erhaltenen Feed-.Teilströme den beiden oder bei Verwendung von mehr als 2 Reaktoren mindestens 2 der Reaktoren unter Zusatz von frischem Feed so zugeführt werden, dass die Temperatur in einem der Reaktoren maximal 20°C höher ist als die in jedem der anderen verwendeten Reaktoren.

2. Verfahren nach Anspruch 1, **dadurch gekennzeichnet, dass** Tₑᵢₙ im Bereich von 20 bis 120°C liegt.

3. Verfahren nach Anspruch 1 oder 2, **dadurch gekennzeichnet, dass** die Temperatur in einem der Reaktoren maximal 10°C höher ist als die in jedem der anderen verwendeten Reaktoren.

4. Verfahren nach den Ansprüchen 1 bis 3, **dadurch gekennzeichnet, dass** der Anteil an Oligomeren im Feed-Strom und in den Feed-Teilströmen 30 Gew.-% nicht übersteigt.

5. Verfahren nach den Ansprüchen 1 bis 4, **dadurch gekennzeichnet, dass** der Feed-Strom und die Feed-Teilströme in kondensierter Form zur Umsetzung gebracht werden.

## Claims

1. A process for preparing oligomers of alkenes having from 4 to 8 carbon atoms from a feed stream comprising such alkenes or hydrocarbon streams in which such alkenes are present over a nickel-containing, heterogeneous catalyst in n successive adiabatically operated reactors, where n is 2 or an integer greater than 2, at from 30 to 280°C and pressures of from 1 to 300 bar, where the feed stream has a temperature Tᵢₙ when it enters the first reaction zone, experiences a temperature increase in each reaction zone and, if this temperature increase is more than Tᵢₙ + 20°C, is brought to a temperature in the range Tᵢₙ± 20°C before it enters a subsequent reaction zone, wherein the feed stream is divided and the feed substreams obtained in this way are fed to the 2 reactors, or if more than 2 reactors are used to at least 2 of the reactors, with addition of fresh feed in such a way that the temperature in one of the reactors is at most 20°C higher than that in each of the other reactors used.

2. The process according to claim 1, wherein Tᵢₙ is in the range from 20 to 120°C.

3. The process according to claim 1 or 2, wherein the temperature in one of the reactors is at most 10°C higher than that in each of the other reactors used.

4. The process according to any of claims 1 to 3, wherein the proportion of oligomers in the feed stream and in the feed substreams does not exceed 30% by weight.

5. The process according to any of claims 1 to 4, wherein the feed stream and the feed substreams is/are reacted in condensed form.

## Revendications

1. Procédé de fabrication d'oligomères d'alcènes contenant 4 à 8 atomes de carbone à partir d'un courant d'alimentation de tels alcènes ou de courants d'hydrocarbures contenant de tels alcènes sur un catalyseur hétérogène contenant du nickel dans n réacteurs successifs exploités de manière adiabatique, n représentant 2 ou un nombre entier supérieur à 2, à des températures de 30 à 280 °C et des pressions de 1 à 300 bars, le courant d'alimentation entrant avec une température Tₑᵢₙ dans la première zone de réaction, subissant une élévation de la température dans chaque zone de réaction et, si cette élévation de la température est supérieure à Tₑᵢₙ + 20 °C, étant ajusté à une température dans l'intervalle Tₑᵢₙ ± 20 °C avant l'entrée dans une zone de réaction ultérieure, **caractérisé en ce que** le courant d'alimentation est divisé et les courants partiels d'alimentation ainsi obtenus sont introduits dans les deux ou, lors de l'utilisation de plus de 2 réacteurs, dans au moins 2 des réacteurs avec ajout d'une alimentation fraîche, de manière à ce que la température dans un des réacteurs soit supérieure d'au plus 20 °C à celle dans chacun des autres réacteurs utilisés.

2. Procédé selon la revendication 1, **caractérisé en ce que** Tₑᵢₙ se situe dans la plage allant de 20 à 120 °C.

3. Procédé selon la revendication 1 ou 2, **caractérisé en ce que** la température dans un des réacteurs est supérieure d'au plus 10 °C à celle dans chacun des autres réacteurs utilisés.

4. Procédé selon les revendications 1 à 3, **caractérisé en ce que** la proportion d'oligomères dans le courant d'alimentation et dans les courants partiels d'alimentation ne dépasse pas 30 % en poids.

5. Procédé selon les revendications 1 à 4, **caractérisé en ce que** le courant d'alimentation et les courants partiels d'alimentation sont mis en réaction sous forme condensée.
